# EUROPEAN PATENT SPECIFICATION

(11) **EP 1 227 797 B1**
(45) Date of publication and mention of the grant of the patent: **12.01.2005**
(21) Application number: 00977140.3
(22) Date of filing: 10.11.2000
(51) Int. Cl.: A61K 9/14, A61K 31/427, A61K 31/513, A61P 31/18

(54) **SOLID DISPERSION PHARMACEUTICAL FORMULATIONS**
PHARMAZEUTISCHE FORMULIERUNGEN AUF BASIS FESTER DISPERSIONEN
FORMULATIONS PHARMACEUTIQUES DE DISPERSION SOLIDE

(30) Priority: 12.11.1999 US 439514
(43) Date of publication of application: 07.08.2002
(73) Proprietor: ABBOTT LABORATORIES, Abbott Park, IL 60064-6050 (US)
(72) Inventor: FORT, James, J., Midlothian, VA 23113 (US); KRILL, Steven, L., Chatham, NJ 07928 (US); LAW, Devalina, Libertyville, IL 60048 (US); QIU, Yihong, Gurnee, IL 60031 (US); PORTER, William, R., Vernon Hills, IL 60061 (US); SCHMITT, Eric, A., Libertyville, IL 60048 (US)
(74) Representative: Modiano, Guido, Dr.-Ing.
(86) International application number: PCT/US2000/030910
(87) International publication number: WO 2001/034118

(56) References cited:
- WO-A-95/07696
- B.J. AUNGST ET AL.: "Amphiphilic vehicles improve the oral bioavailability of a poorly soluble HIV protease inhibitor at high doses" INTERNATIONAL JOURNAL OF PHARMACEUTICS, vol. 156, no. 1, 1997, pages 79-88, XP001009732 Amsterdam (NL) cited in the application

## Description

### Technical Field of the Invention

The instant invention relates to the fields of pharmaceutical and organic chemistry, and provides novel solid dispersion pharmaceutical formulations with enhanced bioavailability.

### Background of the Invention

One measure of the potential usefulness of an oral dosage form of a pharmaceutical agent is the bioavailability observed after oral administration of the dosage form. Various factors can affect the bioavailability of a drug when administered orally. These factors include aqueous solubility, drug absorption throughout the gastrointestinal tract, dosage strength, and first pass effect. Aqueous solubility is one of the most important of these factors. When a drug has poor aqueous solubility, attempts are often made to identify salts or other derivatives of the drug which have improved aqueous solubility. When a salt or other derivative of the drug is identified which has good aqueous solubility, it is generally accepted that an aqueous solution formulation of this salt or derivative will provide the optimum oral bioavailability. The bioavailability of the aqueous oral solution formulation of a drug is then generally used as the standard or ideal bioavailability against which other oral dosage forms are measured.

For a variety of reasons, including patient compliance and taste masking, a solid dosage form, such as a capsule or tablet, is usually preferred over a liquid dosage form. However, oral solid dosage forms of a drug generally provide a lower bioavailability than oral solutions of the drug. One goal of the development of a suitable solid dosage form is to obtain a bioavailability of the drug that is as close as possible to the ideal bioavailability demonstrated by the oral aqueous solution formulation of the drug.

An alternative dosage form is a solid dispersion. The term solid dispersion refers to the dispersion of one or more active ingredients in an inert carrier or matrix at solid state prepared by the melting (or fusion), solvent, or melting-solvent methods. (Chiou and Riegelman, *Journal of Pharmaceutical Sciences, 60*, 1281 (1971)). The dispersion of a drug or drugs in a salid diluent by mechanical mixing is not included in this category. Solid dispersions may also be called solid- state dispersions.

Retroviral protease inhibiting compounds are useful for inhibiting HIV proteases *in vitro* and in *vivo,* and are useful for inhibiting HIV (human immunodeficiency virus) infections and for treating AIDS (acquired immunodeficiency syndrome). HIV protease inhibiting compounds typically are characterized by having poor oral bioavailability. Examples of HIV protease inhibiting compounds include 2S,3S,5S)-5-(N-(N-((N-methyl-N-((2-isopropyl-4-thiazolyl)methyl)amino)carbonyl)L-valinyl)amino-2-(N-((5-thiazolyl)methoxy-carbonyl)-amino)-1,6-diphenyl-3-hydroxyhexane (ritonavir); (2S, 3S, 5S)-2-(2,6-Dimethylphenoxyacetyl) amino-3-hydroxy-5-[2S-(1-tetrahydro-pyrimid-2-onyl)-3-methy 1 butanoyl] -amino-1,6-diphenylhexane (ABT-378); N-(2(R)-hydroxy-1 (S)-indanyl)-2(R)-phenylmethyl -4(S)-hydroxy-5-(1-(4-(3-pyridylmethyl)-2(S)-N'-(t-butylcar boxamido)-piperazinyl))-pentaneamide(indinavir); N-tert-butyl-decahydro-2-[2(R)-hydroxy-4-phenyl-3(S)-[[N-(2-quinolylcarbonyl)-L-asparaginyl]amino]butyl]-(4aS,8aS)-iso quinoline-3(S)-carboxamide (saquinavir); 5(S)-Boc-amino-4(S)-hydroxy-6-phenyl-2(R)phenylmethylhexanoyl-(L)-Val-(L)-Phe-morpholin-4-ylamide; 1-Naphthoxyacetyl-beta-methylthio-Ala-(2S,3S)-3-amino-2-hydroxy-4-butanoyl 1,3-thiazolidine-4-t-butylamide; 5-isoquinolinoxyacetyl-beta-methylthio-Ala-(2S,3S)-3-amino-2-hydroxy-4-butanoyl-1,3-thiazolidine-4-t-butylamide; [1S-[1R-(R-),2S*])-N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide; VX-478; DMP323 DMP-450; AG1343 (nelfinavir); BMS 186,318; SC-55389a; BILA 1096 BS; and U-140690, or combinations thereof.

While some drugs would be expected to have good solubility in organic solvents, it would not necessarily follow that oral administration of such a solution would give good bioavailability for the drug.

Polyethylene glycol (PEG) solid dispersion formulations are generally known to improve the dissolution and bioavailability of many compounds. However, Aungst *et al*. has recently demonstrated that this was unable to improve the bioavailability of an HIV protease inhibitor with a cyclic urea structural backbone, called DMP 323 (Aungst *et al.*, International *Journal of Pharmaceutics,* 156, 79 (1997)).

Thus, it would be a significant contribution to the art to provide a solid dispersion pharmaceutical formulation of a retroviral protease inhibitor which is more stable and has enhanced bioavailability.

### Summary of the Invention

The instant invention provides a stable solid dispersion comprising a retroviral protease inhibitor and PEG having improved bioavailability.

Also provided by the instant invention is a pharmaceutical composition comprising a stable solid dispersion as described above with a pharmaceutically acceptable carrier, diluent, or excipient.

Additionally provided by the instant invention is a method for preparing a stable solid dispersion as described above.

The instant invention still further provides a method of treating an HIV infection comprising administering an effective amount of a stable solid dispersion as described above to a mammal in need of such treatment.

### Brief Description of the Figures

Figure 1 illustrates the dispersion of amorphous ABT-538 in PEG 8000.
Figure 2 illustrates the bioavailability of a dispersion of amorphous ABT-538 in PEG 8000.
Figure 3 illustrates the in vivo-in vitro correlation of ABT-538.
Figure 4 illustrates the dissolution of ABT-378.
Figure 5 illustrates the dissolution of nelfinavir.

### Detailed Description of the Invention

This invention pertains to the preparation of solid dispersion systems for protease inhibitors with improved dissolution and oral bioavailability.

A solid (molecular) dispersion comprising an HIV protease inhibiting compound may be prepared by dissolving or dispersing the HIV protease inhibiting compound in a sufficient amount of an organic solvent followed by dispersion into a suitable water soluble carrier. Suitable organic solvents include pharmaceutically acceptable solvents such as methanol, ethanol, or other organic solvents in which the protease inhibitor is soluble. Suitable water soluble carriers include polymers such as polyethylene glycol (PEG), pluronics, pentaeythritol, pentaeythritol tetraacetate, polyoxyethylene stearates, poly-ε-caprolactone, and the like.

The organic solvent (preferably ethanol) may then be evaporated away, leaving the drug dispersed/dissolved in the molten matrix, which is then cooled. The solid matrix has the compound finely dispersed (molecular dispersion) in such a way that dissolution of the drug is maximized, thus improving the bioavailability of a drug exhibiting dissolution rate limited absorption. Ease of manufacturing is also an attribute to this type of formulation. Once the organic solvent is evaporated to yield a solid mass, the mass may be ground, sized, and optionally formulated into an appropriate delivery system. Thus, by improving the dissolution of a poorly water soluble drug, the drug in a suitable carrier may be filled into a gelatin capsule as a solid, or the matrix may potentially be compressed into a tablet.

The delivery system of the present invention results in increased solubility and bioavailability, and improved dissolution rate of the HIV protease inhibiting compound.

Other pharmaceutically-acceptable excipients may be added to the formulation prior to forming the desired final product. Suitable excipients include lactose, starch, magnesium stearate, or other pharmaceutically-acceptable fillers, diluents, lubricants, disintegrants, and the like, that might be needed to prepare a capsule or tablet.

The resulting composition comprising the HIV protease inhibiting compound may be dosed directly for oral administration, diluted into an appropriate vehicle for oral administration, filled into capsules, or made into tablets for oral administration, or delivered by some other means obvious to those skilled in the art. The composition can be used to improve the oral bioavailability and solubility of said HIV protease inhibiting compound.

Total daily dosing of HIV protease inhibitors may be administered to a human in single or divided doses in amounts, for example, from 0.001 to 1000 mg/kg body weight daily, but more usually 0.1 to 50 mg/kg body weight daily. Dosage unit compositions may contain such amounts of submultiples thereof to make up the daily dose. It will be understood, however, that the specific dose level for any particular patient will depend upon a variety of factors including the age, body weight, general health, sex, diet, time of administration, rate of excretion, drugs administered in combination and the severity of the particular disease undergoing therapy.

ABT-538 (ritonavir) was preferably used as the HIV protease inhibitor in the instant invention. Additionally, two other protease inhibitors, ABT-378 and nelfinavir mesylate, were tested in solid dispersions to demonstrate the improved dissolution which can be achieved with this system.

One aspect of the instant invention provides a solid dispersion of a compound of formula I 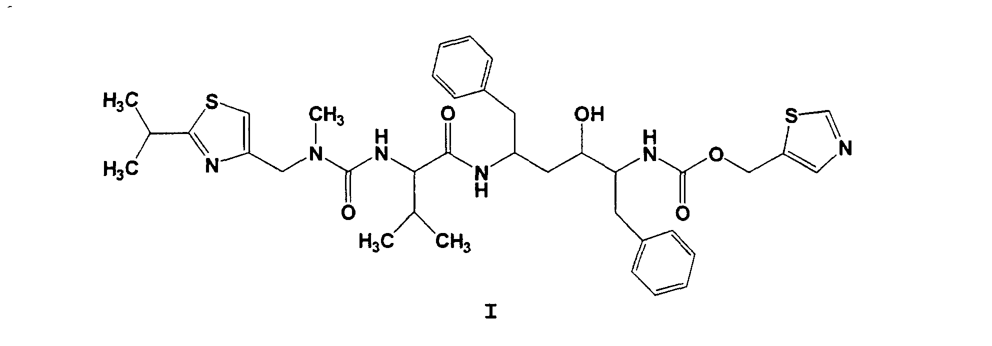 A compound of formula I is an HIV protease inhibitor marketed by Abbott Laboratories under the tradename Norvir® , with the common name ritonavir [(2S,3S,5S)-5-(N-(N-((N-methyl-N-((2-isopropyl-4-thiazolyl)-methyl)amino)carbonyl)-L-valinyl)amino-2-(N-((5-thiazolyl) methoxy-carbonyl) -amino) -1,6-diphenyl-3-hydroxyhexane]. This and other compounds as well as methods for preparing same are disclosed in U.S. Patent Nos. 5,648,497 and 5,541,206, the disclosures of which are herein incorporated by reference.

Additional HIV protease inhibitors which may be formulated into a solid dispersion include compounds of formula II 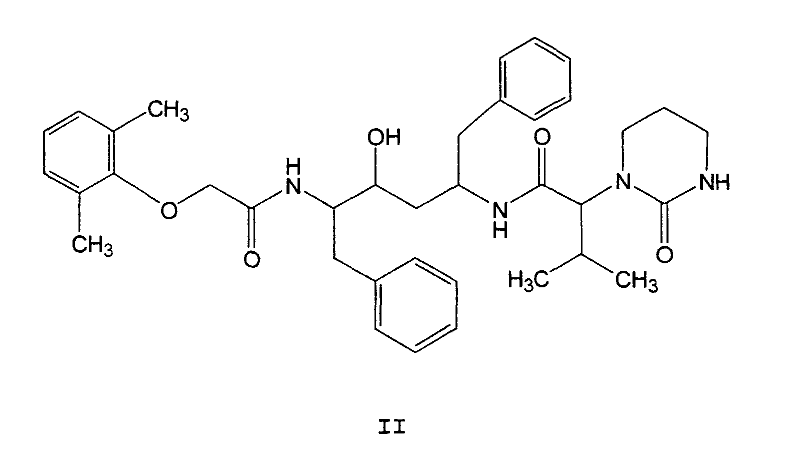 A compound of formula II is known as ABT-378 ((2S,3S,5S)-2-(2,6-dimethylphenoxyacetyl)-amino-3-hydroxy-5-(2S-(1-tetrahydropyrimid-2-onyl)-3-methylbutanoyl)amino-1,6-diphenylhexane). This and other compounds, as well as methods for preparing same, are identified in U.S. Patent No. 5,914,332, the disclosure of which is herein incorporated by reference.

A compound of formula III provided hereinbelow is known as nelfinavir mesylate (marketed under the tradename Viracept® by Agouron Pharmaceuticals, Inc. in La Jolla, CA), and is another HIV protease inhibitor which may be formulated into a solid dispersion.

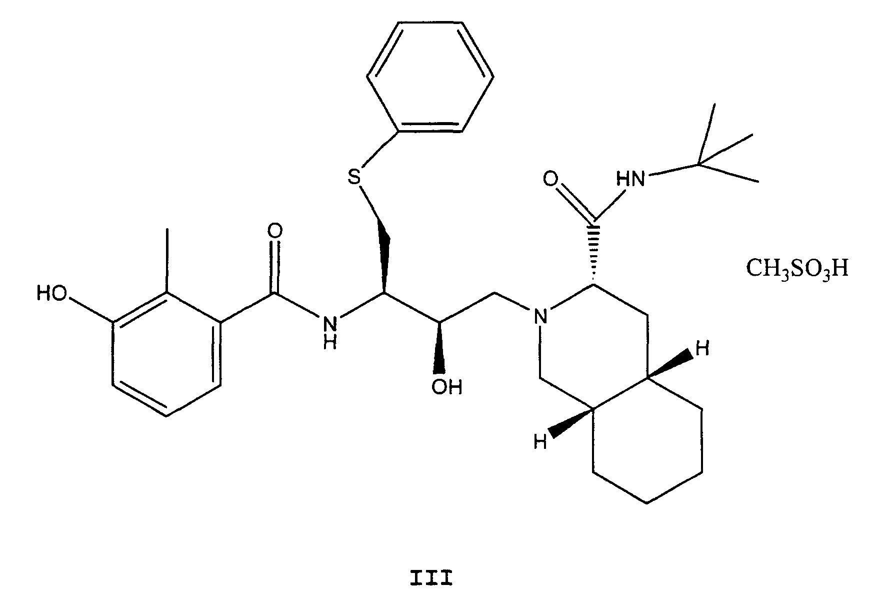

The following Examples are provided to further illustrate the present invention.

### EXAMPLES

### Example 1

### Dispersion Preparations

### A. Ritonavir (ABT-538) Dispersion Preparation:

The samples were prepared by dissolving ABT-538 in a small volume of 200 proof ethanol in a 250 ml round bottom flask. The flask was vortexed and then placed in a water bath maintained at 75 °C. The PEG 8000 was added to the hot alcohol solution with continual swirling until the PEG melted. The flask was then attached to a rotary evaporator, immersed in the water bath (75 °C) under vacuum for 15 minutes to remove the ethanol. After the majority of ethanol had evaporated, the flask was immersed in an ice bath for 15 minutes. The contents of the flask were then vacuum dried at room temperature for 6 hours. The solid was transferred to a crystallization dish and placed under vacuum overnight to remove residual ethanol. The material was then ground and sifted. Particles ranging in size from 149 to 420 *µ*m were used for further studies. The drug loads used for these dispersions were 10, 20 and 30% w/w.

### B. ABT-378 Dispersion Preparation:

A 10% dispersion was prepared using an alcoholic solution of ABT-378 (ca. 0.1 g/ml) by the same method as described in section A above.

### C. Nelfinavir mesylate Dispersion Preparation:

Nelfinavir mesylate is available from Agouron Pharmaceuticals, Inc. under the tradename Virucept® .

A 10% dispersion was prepared using an alcoholic solution of nelfinavir (ca. 0.035 g/ml) by the same method as described in section A above.

The potency values of all the dispersions as well as the dissolution sample concentrations were determined via HPLC.

### D. Results:

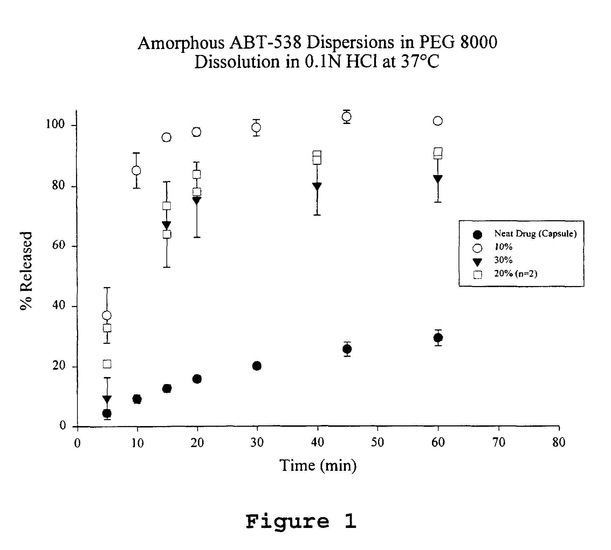

The *in vitro* dissolution data of the ABT-538 dispersions compared with ABT-538 in 0.1*N* HCl (shown in Figure 1, n=3 ± SD unless otherwise indicated) show that the dispersions markedly improved the dissolution rate of the drug. Drug loading decreases the rate of drug release in a rank order. A bioavailability study was conducted in dogs with the above ABT-538 dispersions to elicit the drug load effects in vivo. Eight beagle dogs, obtained from Marshall Research Animals (North Rose, NY), were utilized in this study. The animals were fasted overnight prior to dosing in each period but water was allowed *ad libitum.* Approximately 30 minutes prior to dosing, each dog received a 100 µg/kg subcutaneous dose of histamine. Capsules containing 5 mg/kg of 10, 20 and 30% solid dispersion (formulations A, B and C, respectively) were tested against crystalline drug as a reference in a four-way crossover study.

Each dog received the dose followed by approximately 10 ml of water. A washout period of approximately 1 week was used to separate each dosing period. The plasma samples were analyzed by a method reported by Marsh et al. (Marsh, K.C., Eiden, E. and McDonald, E. Determination of Ritonavir, a new HIV Protease Inhibitor, in Biological Samples Using Reversed-Phase High-Performance Liquid Chromatography. *J. Chromatography B.* 704 (1997) 307-313.)

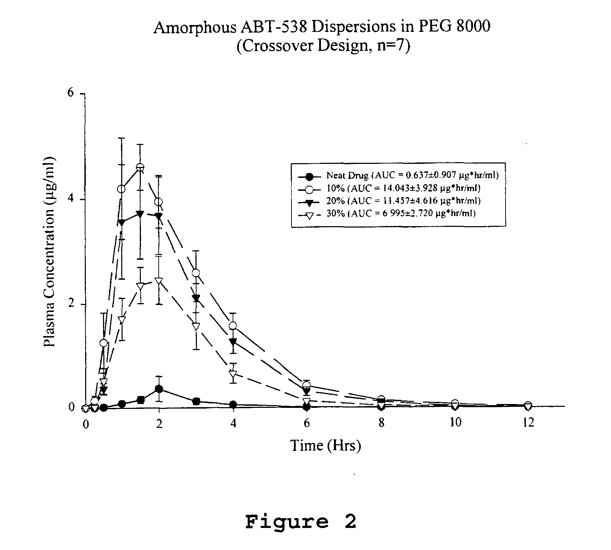

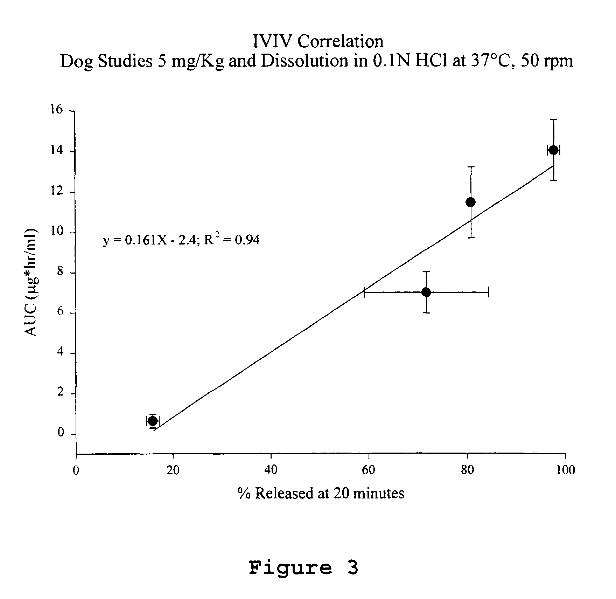

The results of the study are shown in Figure 2. The results show that the solid dispersions improve absorption compared to the reference. An *in vitro* - *in vivo* correlation was established. A plot of the AUC versus the amount dissolved in 20 min, shown in Figure 3, is a straight line, indicating excellent correlation.

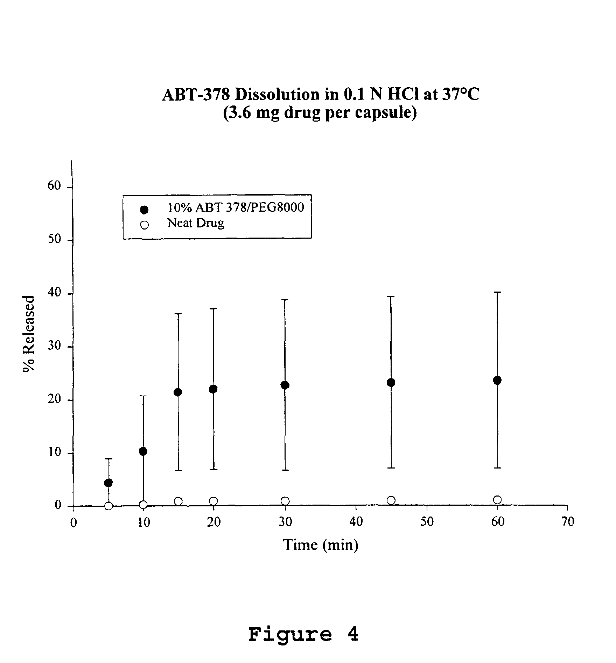

The dissolution properties of the two additional protease inhibitors (ABT-378 and nelfinavir mesylate) were also determined. The in vitro dissolution data (Figure 4) of the ABT-378 dispersion compared with reference clearly shows that the preparation of a dispersion markedly improves dissolution rate of the drug. The variability in the release rate from the dispersion is due to the fact that the preparation of these dispersions had not been optimized to completely overcome the wetting problem of the drug. Despite this, the improvements observed are significant [95% confidence intervals shown].

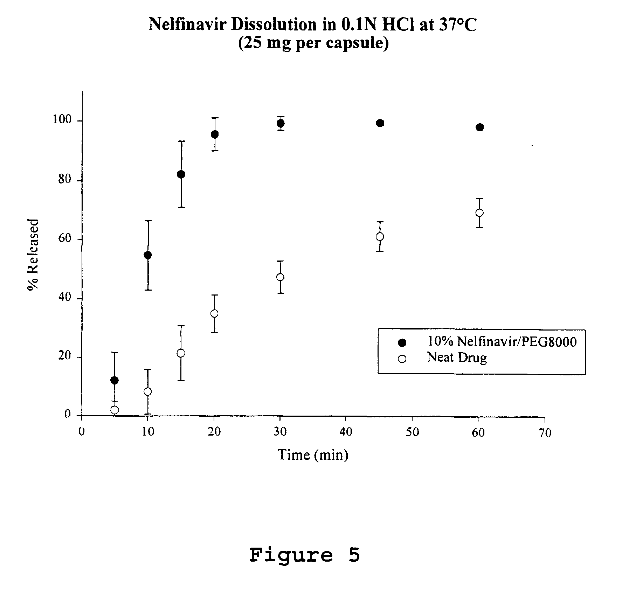

The nelfinavir mesylate solid dispersion also exhibits an improved in vitro dissolution rate compared to the neat drug (Figure 5).

### E. Conclusions:

Solid dispersions of HIV protease inhibitors (for example, ABT-538 (ritonavir), ABT-378, and nelfinavir mesylate) markedly improve the dissolution rate of these drugs. This improvement of dissolution rate is reflected in the improvement of bioavailability. An excellent *in vivo - in vitro* correlation established for the dispersions suggests that the *in vitro* dissolution reflects *in vivo* bioavailability for these systems.

### Example 2

### Stability of Dispersion in Molten PEG 8000

The stability of the dispersion of ABT-538 in PEG 8000 in the molten state at 70 °C was examined. Individual approximately 5 mg quantities of the dispersion (aged for 6 weeks at room temperature) were placed in 4 ml glass vials. These vials, with the exception of the initial time point, were placed in a
70 °C oven which was sampled at pre-determined intervals, chilled in ice water and placed in the freezer until HPLC analysis. After all samples were collected, they were analyzed for ABT-538 content by HPLC. The HPLC system consisted of a Hitachi AS 4000 autosampler, SP 8800 ternary pump. Applied Biosystems 783 detector, and PE Nelson Data acquisition system. Other chromatographic details included a Regis Little Champ 5 cm C-18 column, a mobile phase consisting of an aqueous solution of 0.1% trifluoroacetic acid in 10 mM aqueous tetramethyl ammonium perchlorate (TMAP)/acetonitrile/methanol (55/40/5). The flow rate was 1 ml/minute, the wavelength of detection was 205 nm, and the injection volume was 100 *µ*l. Standard curves of peak area of ABT-538 vs. concentration in the range of interest were compared with experimentally obtained area counts.

### Example 3

### Additional Protocol For Oral Bioavailability Studies

Dogs (beagle dogs, mixed sexes, weighing 7-14 kg) are fasted overnight prior to dosing, but are permitted water *ad libitum.* Each dog receives a 100 *µ*g/kg subcutaneous dose of histamine approximately 30 minutes prior to dosing. Each dog receives a single solid dosage form corresponding to a 5 mg/kg dose of the drug. The dose is followed by approximately 10 milliliters of water. Blood samples are obtained from each animal prior to dosing and at 0.25, 0.5, 1.0, 1.5, 2, 3, 4, 6, 8, 10 and 12 hours after drug administration. The plasma is separated from the red cells by centrifugation and frozen (- 30 °C) until analysis. The concentrations of parent drug is determined by reverse phase HPLC with low wavelength UV detection following liquid-liquid extraction of the plasma samples. The parent drug area under the curve is calculated by the trapezoidal method over the time course of the study. The absolute bioavailability of each test composition is calculated by comparing the area under the curve after oral dosing to that obtained from a single intravenous dose. Each capsule or capsule composition is evaluated in a group containing at least six dogs. The values reported are averages for each group of dogs.

## Claims

1. A pharmaceutical composition comprising a solid dispersion of an HIV protease inhibitor or combination of HIV protease inhibitors and a water soluble carrier wherein the inhibitor or inhibitors are selected from the group consisting of: (2S, 3S, 5S)-5- (N- (N- ( (N-methyl-N- ( (2-isopropyl-4- thiazolyl) methyl) amino) carbonyl) L-valinyl) amino-2- (N- ( (5- thiazolyl) methoxy-carbonyl)-amino)-1, 6-diphenyl-3-hydroxyhexane (ritonavir); (2S, 3S, 5S)-2- (2,6-Dimethylphenoxyacetyl) amino-3-hydroxy-5-[2S-(1-tetrahydro-pyrimid-2-onyl)-3-methy 1 butanoyl] amino-1, 6-diphenylhexane (ABT-378); N-(2 (R)-hydroxy-1 (S)-indanyl)-2 (R)-phenylmethyl -4 (S)-hydroxy-5- (1- (4- (3-pyridylmethyl)-2 (S)-N'- (t-butylcar boxamido)-piperazinyl))-pentaneamide (indinavir); N-tert-butyl-decahydro-2- [2 (R)-hydroxy-4-phenyl-3 (S)- [ [N- (2 -quinolylcarbonyl)-L-asparaginyl] amino] butyl]- (4aS, 8aS)-iso quinoline-3 (S)-carboxamide (saquinavir); 5(S)-Boc-amino-4 (S)-hydroxy-6-phenyl-2 (R)-phenylmethylhexanoyl- (L)-Val- (L)-Phe-morpholin-4-ylamide; 1-Naphthoxyacetyl-beta-methylthio-Ala- (2S, 35)-3-amino-2-hydroxy-4-butanoyl 1,3-thiazolidine-4-t-butylamide; 5-isoquinolinoxyacetyl-beta-methylthio-Ala- (2S, 35)-3-amino-2-hydroxy-4-butanoyl-1 , 3-thiazolidine-4-t-butylamide; [1S- [1R- (R-), 2S*])-N¹- [3- [ [ [ (1, 1- dimethylethyl) amino] carbonyl] . (2-methylpropyl) amino]-2-hydroxy-1-(phenylmethyl) propyl]-2-[(2-quinolinylcarbonyl) amino]-butanediamide ; VX-478; DMP-450; AG1343 (nelfinavir); BMS 186,318; SC-55389a; BILA 1096 BS; U-140690.

2. The composition of Claim 1 wherein said water soluble carrier is polyethylene glycol (PEG).

3. The composition of Claim 2 wherein said HIV protease inhibitor is selected from the group consisting of: (2S, 3S, SS)-5-(N-(N-((N-methyl-N-((2-isopropyl-4-thiazolyl)methyl)amino) carbonyl)L-valinyl)-amino-2-(N-((5- thiazolyl) methoxy- carbonyl) amino)-1,6 diphenyl-3hydroxyhexane (ritonavir); (2S, 3S, 5S)-2- (2, 6Dimethylphenoxyacetyl) amino-3-hydroxy-5-[2S-(1-tetrahydro-pyrimid-2-onyl)-3-methy l butanoyl] amino-1, 6-diphenylhexane (ABT-378); N- (2 (R)-hydroxy-1 (S)-indanyl)-2 (R)-phenylmethyl -4 (S)-hydroxy-5- (1- (4- (3-pyridylmethyl)-2 (S)-N'- (t-butylcar boxamido)-piperazinyl))-pentaneamide (indinavir); N-tert-butyl-decahydro-2- [2 (R)-hydroxy-4-phenyl-3 (S)- [ [N- (2 -quinolylcarbonyl)-L-asparaginyl] amino] butyl]- (4aS, 8aS)-iso quinoline-3 (S)-carboxamide (saquinavir); 5 (S)-Boc-amino-4 (S)-hydroxy-6-phenyl-2 (R)phenylmethylhexanoyl- (L)-Val- (L)-Phe-morpholin-4-ylamide; 1-Naphthoxyacetyl-beta-methylthio-Ala- (2S, 3S)3-amino-2-hydroxy-4-butanoyl 1, 3-thiazolidine-4t-butylamide; 5-isoquinolinoxyacetyl-beta-methylthio-Ala- (2S, 3S)-3-amino-2-hydroxy-4-butanoyl-1, 3-thiazolidine-4-t-butylamide; [1S- [1R- (R-), 2S*])-N¹- [3- [[[(1, 1- dimethylethyl) amino] carbonyl] (2-methylpropyl) amino]-2-hydroxy-1-(phenylmethyl) propyl]-2-[(2-quinolinylcarbonyl) amino]-butanediamide; VX-478; DMP-450; AG1343 (nelfinavir); BMS 186,318; SC-55389a; BILA 1096 BS; U-140690, or combinations thereof.

4. The composition of Claim 2 wherein said HIV protease inhibitor is (2S, 3S, 5S)-5- (N- (N-( (N-methyl-N- ( (2- isopropyl-4-thiazolyl) methyl) amino) carbonyl) L-valinyl) amino-2- (N-((5-thiazolyl) methoxy-carbonyl)-amino) 1 , 6-diphenyl-3-hydroxyhexane (ritonavir).

5. The composition of Claim 2 wherein said HIV protease inhibitor is (2S, 3S, 5S)-2- (2, 6 Dimethylphenoxyacetyl) amino-3-hydroxy-5- [2S- (1-tetrahydro- pyrimid-2-onyl)-3-methyl-butanoyl] amino-1, 6-diphenylhexane (ABT-378).

6. The composition of Claim 2 wherein said HIV protease inhibitor is a combination of (2S, 3S, 5S)-5- (N- (N- ( (N-methyl-N- ( (2-isopropyl-4- thiazolyl) methyl) amino) carbonyl) L-valinyl) amino-2- (N- ( (5- thiazolyl) methoxy-carbonyl)-amino)-1,6-diphenyl-3 hydroxyhexane (ritonavir) and (2S, 3S, 5S)-2- (2, 6 Dimethylphenoxyacetyl) amino-3-hydroxy-5- [2S- (1-tetrahydro-pyrimid-2-onyl)-3-methyl butanoyl] amino-1, 6-diphenylhexane (ABT-378).

7. The composition of Claim 2 wherein said solid dispersion is encapsulated in a hard gelatin capsule.

8. The composition of Claim 2 wherein said solid dispersion is compressed into a tablet.

9. The composition of Claim 1 further comprising an additive or a mixture of additives independently selected from the group consisting of pharmaceutically acceptable surfactants and antioxidants.

10. A method of preparing a composition of Claim 1 which comprises:
a) dissolving an HIV protease inhibitor into an organic solvent to form a solution;
b) adding a water soluble carrier to said solution to form a mixture;
c) optionally flash evaporating said solvent;
d) optionally drying the resulting residue remaining after evaporation;
e) optionally grinding and sieving the solid dispersion to obtain a resultant product.

11. The method of Claim 10 additionally comprising encapsulating the solid dispersion in a hard gelatin capsule.

12. The method of Claim 10 additionally comprising compressing said solid dispersion into a tablet.

13. The method of Claim 10 wherein said HIV protease inhibitor is (2S, 3S, SS)-S-(N-(N-((N-methyl-N-((2-isopropyl- 4-thiazolyl) methyl) amino) carbonyl) L-valinyl) amino-2- (N- ( (5-thiazolyl) methoxy-carbonyl)-amino)-1, 6-diphenyl-3-hydroxyhexane (ritonavir).

14. The method of Claim 10 wherein said HIV protease inhibitor is (2S, 3S, 5S)-2- (2, 6 Dimethylphenoxyacetyl) amino-3-hydroxy-5- [2S- (1-tetrahydro- pyrimid-2-onyl)-3-methyl butanoyl] amino-1, 6-diphenylhexane (ABT-378).

15. The method of Claim 10 wherein said organic solvent is ethanol.

16. The method of Claim 10 wherein said water soluble carrier is polyethylene glycol (PEG).

17. Use of a solid dispersion of Claim 1 for manufacturing a medicament for treating an HIV infection by administration of an effective amount.

18. The use of Claim 17 wherein said HIV protease inhibitor is selected from the group consisting of (2S, 3S, 5S)-5- (N- (N- ( (N-methyl-N- ( (2-isopropyl-4- thiazolyl) methyl) amino) carbonyl) L-valinyl) amino-2- (N- ( (5- thiazolyl) methoxy-carbonyl)-amino)-1, 6-diphenyl-3hydroxyhexane (ritonavir) and (2S, 3S; 5S)-2- (2, 6 Dimethylphenoxyacetyl) amino-3-hydroxy-5- [2S- (1-tetrahydro- pyrimid-2-onyl)-3-methyl butanoyl] amino-1, 6-diphenylhexane (ABT-378).

19. The use of Claim 17 wherein said HIV protease inhibitor is a combination of (28, 3S, 5S)-5-(N-(N-((N- methyl-N- ( (2-isopropyl-4-thiazolyl) methyl) amino) carbonyl) L-valinyl) amino-2-(N- ( (5-thiazolyl) methoxy-carbonyl)-amino)-1, 6-diphenyl-3-hydroxyhexane (ritonavir) and (2S, 3S, 5S)-2-(2, 6 Dimethylphenoxyacetyl) amino-3-hydroxy-5-[2S-(1-tetrahydro- pyrimid-2-onyl)-3-methyl butanoyl] amino-1, 6-diphenylhexane (ABT -378).

## Patentansprüche

1. Eine pharmazeutische Zusammensetzung, die eine Feststoffdispersion eines HIV Proteaseinhibitors oder einer Kombination von HIV Proteaseinhibitoren und eines wasserlöslichen Trägers umfaßt, worin der Inhibitor oder die Inhibitoren gewählt sind aus der Gruppe bestehend aus: (2S,3S, 5S)-5-(N-(N-((N-Methyl-N-((2-isopropyl-4-thiazolyl)methyl)amino)carbonyl)L-valinyl)amino-2-(N-((5-thiazolyl)methoxy-carbonyl)-amino)-1, 6-Diphenyl-3-hydroxyhexan (Ritonavir); (2S,3S,5S)-2-(2,6-Dimethylphenoxyacetyl)amino-3-hydroxy-5-[2S-(1-tetrahydro-pyrimid-2-onyl)-3-methylbutanoyl]amino-1, 6-Diphenylhexan (ABT-378); N-(2(R)-Hydroxy-1-(S)-indanyl)-2-(R)-phenylmethyl-4-(S)-hydroxy-5-(1-(4-(3-pyridylmethyl)-2-(S)-N'-(t-butylcarboxamido)-piperazinyl))-pentanamid (Indinavir); N-tert-Butyl-decahydro-2-[2-(R)-hydroxy-4-phenyl-3(S)-[[N-(2-chinolylcarbonyl)-Lasparaginyl]amino]butyl]-(4aS,8aS)-isochinolin-3(S)-carboxamid (Saquinavir); 5(S)-Boc-amino-4-(S)-hydroxy-6-phenyl-2-(R)-phenylmethylhexanoyl- (L)-Val-(L)-Phe-morpholin-4-ylamid; 1-Naphthoxyacetyl-beta-methylthio-Ala-(2S,3S)-3-Amino-2-hydroxy-4-butanoyl 1,3-Thiazolidin-4-t-butylamid; 5-Isochinplinoxyacetylbeta-methylthio-Ala-(2S,3S)-3-amino-2-hydroxy-4-butanoyl-1, 3-Thiazolidin-4-t-butylamid; [1S-[1R-(R-),2S*])-N¹-[3-[[[(1,1-Dimethylethyl)amino]carbonyl] (2-methylpropyl)amino]-2-hydroxy-1-(phenylmethyl)propyl]-2-[(2-chinolinylcarbonyl)amino]-butandiamid; VX-478; DMP-450; AG1343 (Nelfinavir); BMS 186,318; SC-55389a; BILA 1096 BS; U-140690.

2. Die Zusammensetzung gemäß Anspruch 1, worin der wasserlösliche Träger Polyethylenglykol (PEG) ist.

3. Die Zusammensetzung gemäß Anspruch 2, worin der HIV Proteaseinhibitor gewählt ist aus der Gruppe bestehend aus: (2S, 3S,5S)-5-(N-(N-((N-Methyl-N-((2-isopropyl-4-thiazolyl)methyl)amino)carbonyl)L-valinyl)-amino-2-(N-((5-thiazolyl)methoxy-carbonyl)amino)-1,6-diphenyl-3-hydroxyhexan (Ritonavir); -(2S,3S,5S)-2-(2,6-Dimethylphenoxyacetyl)amino-3-hydroxy-5-[2S(1-tetrahydro-pyrimid-2-onyl)-3-methylbutanoyl]amino-1,6-diphenylhexan (ABT-378); N-(2(R)-Hydroxy-1(S)-indanyl)-2(R)-phenylmethyl-4(S)-hydroxy-5-(1-(4-(3-pyridylmethyl)-2-(S)-N'-(t-butylcarboxamido)piperazinyl))-pentanamid (Indinavir); N-tert-Butyl-decahydro-2-[2(R)-hydroxy-4-phenyl-3-(S)-[[N-(2-chinolylcarbonyl)-L-asparaginyl] amino]butyl]-(4aS, 8aS)-isochinolin-3(S)-carboxamid (Saquinavir); 5-(S)-Boc-amino-4-(S)-hydroxy-6-phenyl-2-(R)phenylmethylhexanoyl-(L)-Val-(L)-Phe-morpholin-4-ylamid; 1-Naphthoxyacetyl-beta-methylthio-Ala- (2S,3S)-3-amino-2-hydroxy-4-butanoyl-1,3-thiazolidin-4t-butylamid; 5-Isochinolinoxyacetyl-beta-methylthio-Ala-(2S,3S)-3-amino-2-hydroxy-4-butanoyl-1,3-thiazolidin-4-t-butylamid; [1S-[1R-(R-), 2S*])-N¹-[3-[[[(1,1-dimethylethyl)amino]carbonyl](2-methylpropyl)amino]-2-hydroxy-1-(phenylmethyl)propyl]-2-[(2-chinolinylcarbonyl)amino]-butandiamid; VX-478; DMP-450; AG1343 (Nelfinavir); BMS 186,318; SC-55389a; BILA 1096 BS; U-140690 oder Kombinationen davon.

4. Die Zusammensetzung gemäß Anspruch 2, worin der HIV Proteaseinhibitor (2S,3S,5S)-5-(N-(N-((N-Methyl-N-((2-isopropyl-4-thiazolyl)methyl)amino)carbonyl)L-valinyl)amino-2-(N-((5-thiazolyl)methoxy-carbonyl)amino)1,6-diphenyl-3-hydroxyhexan (Ritonavir) ist.

5. Die Zusammensetzung gemäß Anspruch 2, worin der HIV Proteaseinhibitor (2S,3S,5S)-2-(2,6-Dimethylphenoxyacetyl)arnino-3-hydroxy-5-[2S-(1-tetrahydropyrimid-2-onyl)-3-methyl-butanoyl]amino-1,6-diphenylhexan (ABT-378) ist.

6. Die Zusammensetzung gemäß Anspruch 2, worin der HIV Proteaseinhibitor eine Kombination von (2S,3S,5S)-5-(N-(N-((N-Methyl-N-((2-isopropyl-4-thiazolyl)methyl)amino)carbonyl)-Lvalinyl)amino-2-(N-((5-thiazolyl)methoxy-carbonyl)-amino)-1,6-diphenyl-3-hydroxyhexan (Ritonavir) und (2S,3S,5S)-2-(2,6-Dimethylphenoxyacetyl)amino-3-hydroxy-5-[2S-(1-tetrahydropyrimid-2-onyl)-3-methyl-butanoyl]amino-1,6-diphenylhexan (ABT-378) ist.

7. Die Zusammensetzung gemäß Anspruch 2, worin die Feststoffdispersion in einer harten Gelatinekapsel einkapselt ist.

8. Die Zusammensetzung gemäß Anspruch 2, worin die Feststoffdispersion in eine Tablette komprimiert ist.

9. Die Zusammensetzung gemäß Anspruch 1, die weiter einen Zusatzstoff oder eine Mischung aus Zusatzstoffen umfaßt, unabhängig gewählt aus der Gruppe bestehend aus pharmazeutisch verträglichen oberflächenaktiven Stoffen und Antioxidantien.

10. Ein Verfahren zur Herstellung einer Zusammensetzung gemäß Anspruch 1, das folgendes umfaßt:
a) Auflösen eines HIV Proteaseinhibitors in einem organischen Lösungsmittel, um eine Lösung zu bilden;
b) Hinzufügen eines wasserlöslichen Trägers zu der Lösung, um eine Mischung zu bilden;
c) Wahlweise Flash-Verdampfen des Lösungsmittels;
d) Wahlweise Trocknen des resultierenden Rückstands, der nach dem Verdampfen zurückbleibt;
e) Wahlweise Zermahlen und Sieben der Feststoffdispersion, um ein resultierendes Produkt zu erhalten.

11. Das Verfahren gemäß Anspruch 10, das zusätzlich das Einkapseln der Feststoffdispersion in eine harten Gelatinekapsel umfaßt.

12. Das Verfahren gemäß Anspruch 10, das zusätzlich das Komprimieren der Feststoffdispersion in eine Tablette umfaßt.

13. Das Verfahren gemäß Anspruch 10, worin der HIV Proteaseinhibitor (2S,3S,5S)-5-(N-(N-((N-Methyl-N-((2-isopropyl-4-thiazolyl)methyl)amino)carbonyl)-L-valinyl)amino-2-(N-((5-thiazolyl)methoxy-carbonyl)-amino)-1,6-diphenyl-3-hydroxyhexan (Ritonavir) ist.

14. Das Verfahren gemäß Anspruch 10, worin der HIV Proteaseinhibitor (2S,3S,5S)-2-(2,6-Dimethylphenoxyacetyl)-amino-3-hydroxy-5-[2S-(1-tetrahydro-pyrimid-2-onyl)-3-methylbutanoyl]amino-1,6-diphenylhexan (ABT-378) ist.

15. Das Verfahren gemäß Anspruch 10, worin das organische Lösungsmittel Ethanol ist.

16. Das Verfahren gemäß Anspruch 10, worin der wasserlösliche Träger Polyethylenglykol (PEG) ist.

17. Verwendung einer Feststoffdispersion gemäß Anspruch 1 zur Herstellung eines Medikaments für die Behandlung einer HIV Infektion durch Verabreichen einer wirksamen Menge.

18. Die Verwendung gemäß Anspruch 17, worin der HIV Proteaseinhibitor gewählt ist aus der Gruppe bestehend aus (2S, 3S,5S)-5-(N-(N-((N-Methyl-N-((2-isopropyl-4-thiazolyl)methyl)amino)carbonyl)-L-valinyl)amino-2-(N-((5-thiazolyl)methoxy-carbonyl)-amino)-1, 6-Diphenyl-3-hydroxyhexan (Ritonavir) und (2S,3S,5S)-2-(2,6-Dimethylphenoxyacetyl)amino-3-hydroxy-5-[2S-(1-tetrahydro-pyrimid-2-onyl)-3-methylbutanoyl]amino-1,6-diphenylhexan (ABT-378).

19. Die Verwendung gemäß Anspruch 17, worin der HIV Proteaseinhibitor eine Kombination aus (2S,3S,5S)-5-(N-(N-((N-Methyl-N-((2-isopropyl-4-thiazolyl)methyl)amino)carbonyl)Lvalinyl)amino-2-(N-((5-thiazolyl)methoxy-carbonyl)-amino)-1,6-diphenyl-3-hydroxyhexan (Ritonavir) und (2S,3S,5S)-2-(2,6-Dimethylphenoxyacetyl)amino-3-hydroxy-5-[2S-(1-tetrahydropyrimid-2-onyl)-3-methyl-butanoyl)amino-1,6-diphenylhexan (ABT-378) ist.

## Revendications

1. Composition pharmaceutique comprenant une dispersion solide d'un inhibiteur de protéase de VIH ou une combinaison d'inhibiteurs de protéase de VIH et un support hydrosoluble où l'inhibiteur ou les inhibiteurs sont choisis dans le groupe consistant en : le (2S,3S,5S)-5-(N-(N-((N-méthyl-N-((2-isopropyl-4-thiazolyl)méthyl)amino)carbonyl)L-valinyl) amino-2-(N-((5-thiazolyl)méthoxy-carbonyl)-amino)-1,6-diphényl-3-hydroxyhexane (ritonavir) ; le (2S,3S,5S)-2-(2,6-diméthylphénoxyacétyl)-amino-3-hydroxy-5-[2S-(1-tétrahydro-pyrimid-2-onyl)-3-méthylbutanoyl]-amino-1,6-diphénylhexane (ABT-378) ; le N-(2(R)-hydroxy-1(S)-indanyl)-2(R)-phénylméthyl-4(S)-hydroxy-S-(1-(4-(3-pyridylméthyl)-2(S)-N'-(t-butylcarboxamido)-pipérazinyl))-pentaneamide (indinavir) ; le N-tert-butyl-décahydro-2-[2(R)-hydroxy-4-phényl-3(S)-[[N-(2-quinolylcarbonyl)-L-asparaginyl]amino]butyl]-(4aS,8aS)-isoquinoléine-3(S)-carboxamide (saquinavir) ; le 5(S)-Boc-amino-4(S)-hydroxy-6-phényl-2(R)-phényl-méthylhexanoyl-(L)-Val-(L)-Phe-morpholin-4-ylamide ; le 1-Naphtoxy-acétyl-beta-méthylthio-Ala-(2S,3S)-3-amino-2-hydroxy-4-butanoyl 1,3-thiazolidine-4-t-butylamide ; le 5-isoquinolinoxyacétyl-beta-méthylthio-Ala-(2S,3S)-3-amino-2-hydroxy-4-butanoyl-1,3-thiazolidine-4-t-butylamide ; le [15-[1R-(R-),2S*])-N¹-[3-[[[(1,1-diméthyléthyl)amino]carbonyl](2-méthylpropyl)amino]-2-hydroxy-1-(phénylméthyl)propyl]-2-[(2-quinolinylcarbonyl)amino]-butanediamide ; VX-478 ; DMP-450 ; AG1343 (nelfinavir) ; BMS 186,318 ; SC-55389a ; BILA 1096 BS ; U-140690.

2. Composition selon la revendication 1 où ledit support hydrosoluble est le polyéthylèneglycol (PEG).

3. Composition selon la revendication 2 où ledit inhibiteur de protéase de VIH est choisi dans le groupe consistant en : le (2S,3S,5S)-5-(N-(N-((N-méthyl-N-((2-isopropyl-4-thiazolyl)méthyl)amino)carbonyl)L-valinyl)-amino-2-(N-((5-thiazolyl)méthoxy-carbonyl)amino)-1,6-diphényl-3-hydroxyhexane (ritonavir) ; le (2S,3S,5S)-2-(2,6-diméthylphénoxyacétyl)-amino-3-hydoxy-5-[2S-(1-tétrahydro-pyrimid-2-onyl)-3-méthyl-1-butanoyl] amino-1,6-diphénylhexane (ABT-378) ; le N-(2(R)-hydroxy-1(S)-indanyl-2 (R)-phénylméthyl-4(S)-hydroxy-5-(1-(4-(3-pyridylméthyl)-2(S)-N'-(t-butylcarboxamido)-pipérazinyl))-pentaneamide (indinavir) ; le N-tert-butyldécahydro-2-[2(R)-hydroxy-4-phényl-3(S)-[[N-(2-quinolylcarbonyl)-L-asparaginyl]amino]butyl]-(4aS,8aS)-isoquinoléine-3(S)-carboxamide (saquinavir) ; le 5(S)-Boc-amino-4(S)-hyroxy-6-phényl-2(R)phénylméthylhexanoyl-(L)-Val-(L)-Phe-morpholin-4-ylamide ; le 1-naphtoxyacétyl-beta-méthylthio-Ala-(2S,3S)3-amino-2-hydroxy-4-butanoyl 1,3-thiazolidine-4t-butylamide ; le 5-isoquinolinoxyacétyl-beta-méthylthio-Ala-(2S,3S)-3-amino-2-hydroxy-4-butanoyl-1,3-thiazolidine-4-t-butylamide ; le [1S-[1R-(R-),2S*])-N¹-[3-[[[(1,1-diméthyléthyl)amino]carbonyl](2-méthylpropyl)amino]-2-hydroxy-1-(phénylméthyl)propyl]-2-[(2-quinolinylcarbonyl)amino]-butanediamide ; VX-478 ; DMP-450 ; AG1343 (nelfinavir) ; BMS 186,318 ; SC-55389a ; BILA 1096 BS ; U-140690 ou leurs combinaisons.

4. Composition selon la revendication 2 où ledit inhibiteur de protéase de VIH est le (2S,3S,5S)-5-(N-(N-((N-méthyl-N-((2-isopropyl-4-thiazolyl)méthyl)amino)carbonyl)L-valinyl)amino-2-(N-((5-thiazolyl)-méthoxy-carbonyl)-amino)1,6-diphényl-3-hydroxyhexane (ritonavir).

5. Composition selon la revendication 2 où ledit inhibiteur de protéase de VIH est le (2S,3S,5S)-2-(2,6-diméthylphénoxyacétyl)amino-3-hydroxy-5-[2S-( 1-tétrahydro-pyrimid-2-onyl)-3-méthylbutanoyl]amino-1,6-diphénylhexane (ABT-378).

6. Composition selon la revendication 2 où ledit inhibiteur de protéase de VIH est une combinaison de (2S,3S,5S)-5-(N-(N-((N-méthyl-N-((2-isopropyl-4-thiazolyl)méthyl)amino)carbonyl)L-valinyl)amino-2-(N-((5-thiazolyl)méthoxy-carbonyl)-amino)-1,6-diphényl-3-hydroxyhexane (ritonavir) et de (2S,3S,5S)-2-(2,6-diméthylphénoxyacétyl)amino-3-hydroxy-5-[2S-(1-tétrahydro-pyrimid-2-onyl)-3-méthylbutanoyl]amino-1,6-diphénylhexane (ABT-378).

7. Composition selon la revendication 2 où ladite dispersion solide est encapsulée dans une capsule de gélatine dure.

8. Composition selon la revendication 2 où ladite dispersion solide est compressée en un comprimé.

9. Composition selon la revendication 1 comprenant en outre un additif ou un mélange d'additifs choisis indépendamment dans le groupe consistant en les tensioactifs et les antioxydants pharmaceutiquement acceptables.

10. Procédé de préparation d'une composition selon la revendication 1 qui comprend :
a) la dissolution d'un inhibiteur de protéase de VIH dans un solvant organique pour former une solution ;
b) l'addition d'un support hydrosoluble à ladite solution pour former un mélange ;
c) éventuellement l'évaporation éclair dudit solvant ;
d) éventuellement le séchage du résidu résultant restant après l'évaporation ;
e) éventuellement le broyage et le tamisage de la dispersion solide pour obtenir un produit résultant.

11. Procédé selon la revendication 10 comprenant en outre l'encapsulation de la dispersion solide dans une capsule de gélatine dure.

12. Procédé selon la revendication 10 comprenant en outre la compression de ladite dispersion solide en un comprimé.

13. Procédé selon la revendication 10 où ledit inhibiteur de protéase de VIH est le (2S,3S,5S)-5-(N-(N-((N-méthyl-N-((2-isopropyl-4-thiazolyl)méthyl)amino)carbonyl)L-valinyl)amino-2-(N-((5-thiazolyl)-méthoxy-carbonyl)-amino)-1,6-diphényl-3-hydroxyhexane (ritonavir).

14. Procédé selon la revendication 10 où ledit inhibiteur de protéase de VIH est le (2S,3S,5S)-2-(2,6-diméthylphénoxyacétyl)amino-3-hydroxy-5-[2S-(1-tétrahydro-pyrimid-2-onyl)-3-méthylbutanoyl]amino-1,6-diphénylhexane (ABT-378).

15. Procédé selon la revendication 10 où ledit solvant organique est l'éthanol.

16. Procédé selon la revendication 10 où ledit support hydrosoluble est le polyéthylèneglycol (PEG).

17. Utilisation d'une dispersion solide selon la revendication 1 pour la production d'un médicament pour traiter une infection à VIH par administration d'une quantité efficace.

18. Utilisation selon la revendication 17 où ledit inhibiteur de protéase de VIH est choisi dans le groupe consistant en le (2S,3S,5S)-5-(N-(N-((N-méthyl-N-((2-isopropyl-4-thiazolyl)méthyl)amino)carbonyl)L-valinyl)amino-2-(N-((S-thiazolyl)méthoxy-carbonyl)-amino)-1,6-diphényl-3-hydroxyhexane (ritonavir) et le (25,3S,5S)-(2,6-diméthylphénoxyacétyl)-amino-3-hydroxy-5-[2S-(1-tétrahydro-pyrimid-2-onyl)-3-méthylbutanoyl]-amino-1,6-diphénylhexane (ABT-378).

19. Utilisation selon la revendication 17 où ledit inhibiteur de protéase de VIH est une combinaison de (2S,3S,5S)-5-(N-(N-((N-méthyl-N-((2-isopropyl-4-thiazolyl)méthyl)amino)carbonyl)L-valinyl)amino-2-(N-((5-thiazolyl)méthoxy-carbonyl)-amino)-1,6-diphényl-3-hydroxyhexane (ritonavir) et de (2S,3S,SS)-2-(2,6-diméthylphénoxyacétyl)amino-3-hydroxy-5-[2S-(1-tétrahydro-pyrimid-2-onyl)-3-méthylbutanoyl]amino-1,6-diphénylhexane (ABT-378).
